# EUROPEAN PATENT SPECIFICATION

(11) **EP 3 116 468 B1**
(45) Date of publication and mention of the grant of the patent: **14.11.2018**
(21) Application number: 15710380.5
(22) Date of filing: 05.03.2015
(51) Int. Cl.: A61K 8/34, A61Q 5/12, A61K 8/81, A61K 8/92

(54) **HAIR CARE SENSORY AGENTS**
SENSORISCHE HAARPFLEGEMITTEL
AGENTS SENSORIELS DE SOINS CAPILLAIRES

(30) Priority: 10.03.2014 US 201461950447 P
(43) Date of publication of application: 18.01.2017
(73) Proprietor: Dow Global Technologies LLC, Midland, MI 48674 (US); Rohm and Haas Company, Philadelphia, PA 19106 (US)
(72) Inventor: CLARK, Thomas P., Midland, Michigan 48674 (US); JOSHI, Kinjalbahen, Collegeville, Pennsylvania 19426 (US); KELLER, Kathleen, Collegeville, Pennsylvania 19426 (US); KRAMER, John W., Midland, Michigan 48674 (US); O'CONNOR, Ying, Collegeville, Pennsylvania 19426 (US); PETERSON, Thomas H., Midland, Michigan 48674 (US); SCHWARTZ, Curtis, Collegeville, Pennsylvania 19426 (US); WAN, Qichun, Midland, Michigan 48674 (US)
(74) Representative: Houghton, Mark Phillip
(86) International application number: PCT/US2015/018918
(87) International publication number: WO 2015/138210

(56) References cited:
- WO-A2-2014/047102
- US-A1- 2007 031 361
- US-A1- 2011 064 683

## Description

### FIELD OF THE INVENTION

This invention relates generally to compositions that are useful as sensory agents in hair care formulations. The compositions contain a polyolefin blend.

### BACKGROUND

Personal care products, particularly hair care products such as leave-on conditioners, require a smooth and silky feel on skin to please consumers. In fact, aesthetics are one of the most important factors in consumer satisfaction. Accordingly, the hair care art has developed sensory agents, such as silicone oils, hard particles (such as Poly(methyl methacrylate) (PMMA) particles and polyethylene (PE) particles), and silicone elastomer gels in order to impart good aesthetics. However, each of the foregoing is associated with certain drawbacks, like insufficient sensory performance, poor conditioning, stability, and texture, or relatively high cost.

One attempt to overcome the problems associated with such sensory agents is disclosed in U.S. Patent No. 6,180,123 in the form of a composition containing a crystalline olefin copolymer having a degree of crystallinity at most equal to 50% dispersed in a liquid fatty phase, and which composition contains at least 2% by weight of the olefin copolymer. Such compositions, however, are disclosed for use in keratinous substances such as binders or cast products and are thus not suitable for use as leave-on conditioners due to insufficient sensory performance.

D2 discloses personal care compositions with ethylene acrylic acid copolymer aqueous dispersions.

Accordingly, there is a continuing need in the art for cost-effective high performance sensory agents, preferably with good manageability, anti-frizz properties, and shine profiles in hair care formulations.

### STATEMENT OF THE INVENTION

We have now found that polyolefin blends, which contain a first metallocene catalyzed polyolefin with a density above 0.90 g/cm³ and a second metallocene catalyzed polyolefin with a density equal to or below 0.90 g/cm³, are highly effective sensory agents in hair care formulations and exhibit significantly better sensory properties compared to previously known sensory agents for hair care compositions.

Accordingly, one aspect of the present invention provides a hair care composition containing: (a) a polyolefin oil blend comprising (i) at least one high density metallocene catalyzed polyolefin with a density greater than 0.90 g/cm³, (ii) at least one low density matellocene catalyzed polyolefin with a density equal to or below 0.90 g/cm³, and (iii) a cosmetically acceptable hydrocarbon oil; (b) a thickener; and (c) a humectant. In certain embodiments, the polyolefin oil blend is present in the composition in an amount of from 0.1 to 1.4 weight % by weight of the composition. In certain embodiments, the high and low density polyolefins in the polyolefin oil blend have an average melt index greater than 7. In certain embodiments, the composition is substantially free of ethylene-acrylic acid copolymer.

### BRIEF DESCRIPTION OF THE FIGURES

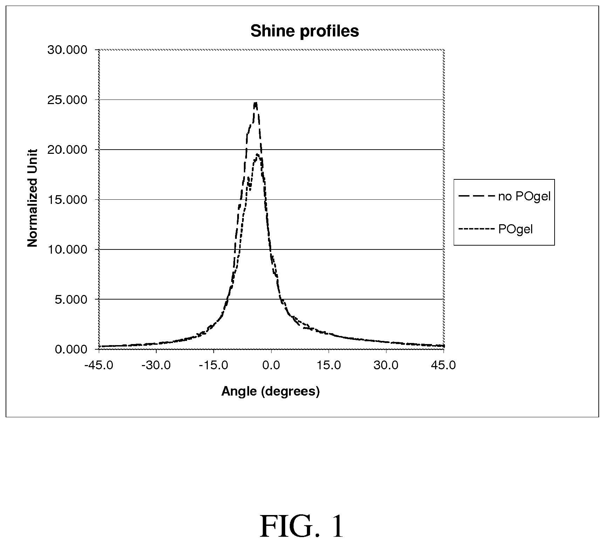

FIG. 1 is a shine profile of two different hair swatches - one treated with a leave-on conditioner containing a polyolefin oil blend in accordance with the present invention, and another with a control leave-on conditioner that does not contain a polyolefin oil blend.

### DETAILED DESCRIPTION

Without wishing to be bound by theory, we have discovered that metallocene catalyzed polyolefins of medium or high density (i.e., polyolefins having a density above 0.90 g/cm³ as measured by ASTM D 792) impart good aesthetic properties, but form unstable gels. In contrast, low density metallocene catalyzed polyolefins (i.e., polyolefins having a density of from 0.86 to 0.90 g/cm³) form stable gels, but impart poor aesthetic properties. Moreover, low molecular weight metallocene catalyzed polyolefins (i.e., polyolefins having a melt index greater than or equal to 8 as measured by ASTM D 1238) demonstrate very good solubility in a hydrocarbon medium and maintain phase homogeneity. In contrast, high molecular weight metallocene catalyzed polyolefins (i.e., polyolefins having a melt index less than 8 as measured by ASTM D 1238) lead to hard gels that are not easily workable in personal care formulations. We have now surprisingly found that compositions comprising a polyolefin oil blend impart improved sensorial and aesthetic properties demonstrating good manageability, anti-frizz properties, and shine profiles in hair care formulations.

As noted above, provided are hair care compositions, preferably leave-on hair conditioners, comprising a polyolefin oil blend, a humectant, and a thickener. The polyolefin oil blend comprises at least one high density metallocene catalyzed polyolefin with a density above 0.90 g/cm³, at least one low density metallocene catalyzed polyolefin with a density equal to or below 0.90 g/cm³, preferably from 0.86 to 0.90 g/cm³, and a cosmetically acceptable hydrocarbon oil. In certain embodiments, the average melt index for the high and low density polyolefins in the polyolefin oil blend is greater than 7, preferably greater than 8, and more preferably greater than 8.5. In certain embodiments, the high density polyolefin has a weight-average molecular weight in a range of from 5,000 to 40,000, preferably 10,000 to 30,000, and more preferably from 20,000 to 28,000. In certain embodiments, the low density polyolefin has a weight-average molecular weight in a range of from 41,000 to 500,000, preferably 70,000 to 90,000, and more preferably from 75,000 to 85,000. In certain preferred embodiments the hair care compositions are substantially free of ethylene-acrylic acid copolymer.

As used herein, the following terms have the designated definitions, unless the context clearly indicates otherwise. "Hair care" relates to compositions to be topically applied to a person, and in particular a person's hair. Examples of hair care compositions include, but are not limited to, shampoos, leave-on and rinse off conditioners, styling gels, and hairsprays. In certain preferred embodiments, the hair care composition is a conditioner, preferably a leave-on hair conditioner. "Cosmetically acceptable" refers to ingredients typically used in personal care compositions, and is intended to underscore that materials that are toxic when present in the amounts typically found in personal care compositions are not contemplated as part of the present invention.

"Metallocene catalyzed polyolefins" are polyolefins produced with a metallocene catalyst. Metallocene catalysis enables control of the polyolefin properties relating to, for example, crystallinity, polymer chain length, and distribution homogeneity of the polymer chain units. Metallocene catalysis also favors uniformity in polymer chains density and length. Suitable metallocene catalysts include, for example, those described in U.S. Patent Nos. 4,701,432, 5,322,728, and 5,272,236. In certain embodiments of the present invention, the metallocene catalyzed polyolefins are polyethylenes produced with a metallocene catalyst. Suitable metallocene catalyzed polyethylenes are available from, for example, The Dow Chemical Company under the trademark AFFINITY or ENGAGE (ethylene/octene copolymers), and from Exxon Chemical Company under the trademark EXACT (ethylene/butene copolymers, ethylene/hexene copolymers, or ethylene/butene/hexene terpolymers). In one embodiment, the metallocene catalyzed polyolefin is at least one of ethylene/octene copolymers, ethylene/butene copolymers, ethylene/hexene copolymers, ethylene/propylene or ethylene/butene/hexene terpolymers, preferably an ethylene octene copolymer. In another embodiment, the metallocene catalyzed polyolefin is a propylene/alpha-olefin copolymer. Suitable propylene/alpha-olefin copolymers include, for example, those described in detail in U.S. Patent Nos. 6,960,635 and 6,525,157. Such propylene/alpha-olefin copolymers are commercially available from The Dow Chemical Company under the trademark VERSIFY, or from ExxonMobil Chemical Company under the trademark VISTAMAXX. Other suitable polyolefins are sold by The Dow Chemical Company under the trademarks AMPLITY, ATTANE, INFUSE, NORDEL, and VLDPE. Other suitable non-limiting examples of commercially available metallocene catalyzed polyethylenes and the melt index and density of each is as shown in Table 1.

**Table 1. Specified Metallocene Catalyzed Polyethylenes**

| **Polyolefin Name** | **Melt Index** | **Density** |
|---|---|---|
| AFFINITY GA 1950 | 500 | 0.874 |
| AFFINITY PL1840G | 1 | 0.909 |
| AMPLIFY EA 103 | 21 | 0.930 |
| AMPLIFY GR 202 | 8 | 0.930 |
| ATTANE 4203 | 0.8 | 0.905 |
| ATTANE 4404G | 4 | 0.904 |
| ENGAGE 8100 | 1 | 0.870 |
| ENGAGE 8130 | 13 | 0.863 |
| ENGAGE 8200 | 5 | 0.870 |
| ENGAGE 8402 | 30 | 0.902 |
| LDPE 4016 | 16 | 0.916 |
| LDPE 640I | 2 | 0.920 |
| LDPE 955I | 35 | 0.923 |
| VERSIFY 2200 | 2 | 0.876 |
| VERSIFY 3200 | 8 | 0.876 |
| VERSIFY 4200 | 25 | 0.876 |

Although ethylene-acrylic acid (EAA) copolymers are known for use in personal care compositions, we have surprisingly found that EAA copolymers deleteriously flocculate and negatively impact the stability of the inventive compositions due to relatively low pH and low surfactant levels. Accordingly, in certain preferred embodiments the inventive compositions described herein are substantially free of EAA. "Substantially free" in this context means less than 3 weight %, preferably less than 1 weight %, more preferably less than 0.1 weight %, and even more preferably zero weight percent present in the composition.

In one embodiment, the at least one high density metallocene catalyzed polyolefin is present in an amount of from 1 to 60 weight %, preferably from 2 to 40 weight %, and more preferably from 5 to 10 weight %, of solids by weight of the polyolefin oil blend. In one embodiment, the at least one low density metallocene catalyzed polyolefin is present in an amount of from 1 to 60 weight %, preferably from 2 to 40 weight %, and more preferably from 5 to 10 weight %, of solids by weight of the polyolefin oil blend. In one embodiment, the ratio of at least one metallocene catalyzed polyolefin with a density above 0.90 g/cm³ to the at least one metallocene catalyzed polyolefin with a density equal to or below 0.90 g/cm³ is between 1:95 and 95:1, preferably between 10:50 and 60:10, and more preferably between 10:40 and 40:10. In certain preferred embodiments, the ratio is 1:1, 1.5:1, 2:1, or 3:1.

The polyolefin oil blend is prepared by shearing the above-described polyolefins in a carrier fluid including, for example, aromatic hydrocarbons, aliphatic hydrocarbons, alcohols, esters, ethers, glycols, carbonates, silicones, glycol ethers at high temperature, above 100°C, preferably from 120°C to 150°C. In one embodiment, the polyolefin oil blend is referred to as an "oil gel," although the polyolefin blend can be powder, pellet/bead, oil gel/ oil paste, or water dispersion.

A variety of cosmetically acceptable hydrocarbon oils are suitable for use in the present invention, and are selected from various carbon chain length oils. In certain embodiments, the hydrocarbon oils include, but are not limited to, C₁₄-C₂₂ hydrocarbon oils. In certain embodiments, the hydrocarbon oil is less than fourteen carbons in length. In certain embodiments, the hydrocarbon oil is greater than twenty-two carbons in length. Suitable hydrocarbon oils include, for example, those sold under the trademarks LILAC, GEMSEAL 25, GEMSEAL 40, PERMETHYL 101A, PERMETHYL 99A, SILKFLO 364 NF, SILKFLO 366 NF, FANCOL POLYISO 200-CG, FANCOL POLYISO 300-CG, FANCOL POLYISO 450-CG, FANCOL POLYISO 800-CG, PANALANE L-14E, PURESYN 2, PURESYN 4, or RITADECENE 20. In certain embodiments, the hydrocarbon oil is present in an amount of from 40 to 98 weight %, preferably 60 to 94 weight %, and more preferably from 80 to 90 weight %, by weight of the polyolefin oil blend.

In certain embodiments, the polyolefin oil blend (containing at least one high density metallocene catalyzed polyolefin, at least one low density metallocene catalyzed polyolefin, and at least one cosmetically acceptable hydrocarbon oil) is present in an amount of from 0.1 to 1.4 weight %, preferably 0.3 to 1.2 weight %, and more preferably 0.5 to 1.0 weight %, by weight of the hair care composition.

In addition to the polyolefin oil blend, the hair care compositions of the present invention include a thickener/rheology modifier. Thickeners are substances that increase the viscosity of a solution or liquid/solid mixture without substantially modifying its other properties. Suitable thickeners include, for example, polysaccharides (e.g., xanthan gum, guar gum, starch, and vegetable gum) and cellulosic polymers (e.g., carboxymethyl cellulose (CMC), hydroxymethyl cellulose (HMC), and hydroxypropyl methyl cellulose (HPMC)). Certain preferred thickeners include, for example, hydrophobically modified cross-linked acrylate copolymers (e.g., those sold by Lubrizol under the trademark CARBOPOL ULTREZ 21). In certain embodiments, the thickener is present in an amount of from 0.1 to 1.0 weight %, preferably from 0.2 to 0.7 weight %, and more preferably from 0.3 to 0.5 weight %, by weight of the hair care composition.

The hair care compositions of the present invention also include a humectant to prevent the loss of moisture. Suitable humectants include, for example, glycerin, sorbitol, monoglycerides, lecithins, glycolipids, fatty alcohols, fatty acids, polysaccharides, sorbitan esters, polysorbates (e.g., Polysorbate 20, Polysorbate 40, Polysorbate 60, and Polysorbate 80), diols (e.g., propylene glycol), diol analogs, triols, triol analogs, polymeric polyols, and mixtures thereof. In certain embodiments, the humectant is present in an amount of from 1 to 20 weight %, preferably 2 to 15 weight %, and more preferably 5 to 10 weight %, by weight of the hair care composition.

In addition to the polyolefin blend, thickener, and humectant, the hair care compositions also contain water in an amount of from 85 to 95 weight %, preferably from 60 to 80 weight %, and more preferably from 50 to 70 weight %, by weight of the hair care composition.

In certain embodiments, the hair care composition contains excipients, such as additional emollients (e.g., hydrocarbon oils, esters, natural oils, or silicones), waxes, sensory modifiers, preservatives/antioxidants/chelating agents, pH adjusting agents/buffers/neutralizing agents, sunscreen actives, vitamins, proteins/amino acids, plant extracts, natural ingredients, bio-actives, fragrances/perfumes, penetrants, polymers/resins/hair fixatives/film formers, surfactants/detergents/emulsifiers/opacifying agents, volatiles/propellants/solvents/carriers, liquid vehicles/solvents/carriers, salts, antistatic agents, anti-frizz agents, antidandruff agents, hair waving/straightening agents, absorbents, colorants, hard particles, conditioning agents, and other silicones.

Some of the embodiments of the invention will now be described in detail in the following Examples.

### EXAMPLES

### Example 1

### Preparation of Example Polyolefin Oil Blend 1

A polyolefin oil blend in the form of a polyolefin oil gel was prepared by combining 6.25 grams of AFFINITY GA 1950, 6.25 grams of AFFINITY PL1840 G, and 87.5 grams of LILAC oil (C₁₄-C₂₂ hydrocarbon oil) in a glass container. The container was placed on a hot plate and set up as a closed system with nitrogen filled on top of the surface. The mixture was mixed with an overhead stirrer at a speed of about 100-200 rpm at a temperature of about 150°C for about 1 hour until all of the solids are melted. The mixture was then cooled while mixing until it reached a temperature of about 85°C.

### Example 2

### Preparation of Example Polyolefin Oil Blend 2

A polyolefin oil blend in the form of a polyolefin oil gel was prepared by combining 4.17 grams of AFFINITY GA 1950, 4.17 grams of LDPE 9551, 4.17 grams of ATTANE 4404G, and 87.5 grams of LILAC oil (C₁₄-C₂₂ hydrocarbon oil) in a glass container. The container was placed on a hot plate and set up as a closed system with nitrogen filled on top of the surface. The mixture was mixed with an overhead stirrer at a speed of about 100-200 rpm at a temperature of about 150°C for about 1 hour until all of the solids are melted. The mixture was then cooled while mixing until it reached a temperature of about 85°C.

### Example 3

### Preparation of a Leave-On Hair Conditions Including a Polyolefin Oil Blend

A leave-on hair conditioner including the polyolefin oil blend prepared in Example 1 above was prepared according to the formulation in Table 2.

**Table 2. Example Leave-On Hair Conditioner Formulation Including Polyolefin Oil Blend**

| **Phase** | **Component** | **INCI** | **Weight %** |
|---|---|---|---|
| A | Water | | 91.78 |
| A | ULTREZ 21 | Acrylates/C10-30 Alkyl Acrylate Crosspolymer | 0.3 |
| B | AMP-ULTRA PC 2000 | Aminomethyl Propanol | 0.12 |
| C | RITACHOL 1000 | Cetearyl Alcohol, Polysorbate 60, PEG-150 Stearate, Steareth 20 | 2.0 |
| C | Glycerin | Glycerin | 1.0 |
| C | UCON Fluid LB-1715 | PPG-40 Butyl Ether | 2.0 |
| C | Polyolefin Oil B lend | | 1.0 |
| C | PROCETYL | PPG-5-Ceteth-20 | 1.0 |
| D | NEOLONE MxP | Methylisothiazolinone, Methylparaben, Propylparaben, Phenoxyethanol | 0.8 |

Phase A was heated to about 40°C and stirred for 10 minutes at 300 rpm. Phases A and B were then combined and heated to 85-90°C while stirring continuously at 300 rpm. Phase C was heated separately to 85-95°C and then added to Phases A and B. Phases A, B, and C were then heated at 85-90°C while stirring at 500 rpm for 10 minutes. The mixture was then cooled while stirring at 300 rpm. Phase D was added once the mixture cooled to 45°C.

### Example 4

### Preparation of Control Leave-On Hair Conditioner

A leave-on hair conditioner not including a polyolefin oil blend was prepared according to the formulation in Table 3.

**Table 3. Comparative Leave-On Hair Conditioner Formulation**

| **Phase** | **Component** | **INCI** | **Weight %** |
|---|---|---|---|
| A | Water | | 92.78 |
| A | ULTREZ 21 | Acrylates/C10-30 Alkyl Acrylate Crosspolymer | 0.3 |
| B | AMP-ULTRA PC 2000 | Aminomethyl Propanol | 0.12 |
| C | RITACHOL 1000 | Cetearyl Alcohol, Polysorbate 60, PEG-150 Stearate, Steareth 20 | 2.0 |
| C | Glycerin | Glycerin | 1.0 |
| C | UCON Fluid LB-1715 | PPG-40 Butyl Ether | 2.0 |
| C | PROCETYL | PPG-5-Ceteth-20 | 1.0 |
| D | NEOLONE MxP | Methylisothiazolinone, Methylparaben, Propylparaben, Phenoxyethanol | 0.8 |

Phase A was heated to about 40°C and stirred for 10 minutes at 300 rpm. Phases A and B were then combined and heated to 85-90°C while stirring continuously at 300 rpm. Phase C was heated separately to 85-95°C and then added to Phases A and B. Phases A, B, and C were then heated at 85-90°C while stirring at 500 rpm for 10 minutes. The mixture was then cooled while stirring at 300 rpm. Phase D was added once the mixture cooled to 45°C.

### Example 5

### Hair Manageability Panel Study - Wet/Dry Combing

Four swatches of Brazilian curly hair were obtained from International Hair Importers & Products Inc. The swatches were washed with a 10% solution of Tergitol 15 S-9 and towel dried to remove excess water from the hair. 0.5 grams of a different leave-on conditioning composition was applied to each of the first two swatches: (1) the inventive leave-on conditioner as prepared in Example 3 above; and (2) the control leave-on conditioner as prepared in Example 4 above. Each conditioner was applied to its respective swatch by rubbing into the hair for 30 seconds. No conditioner was applied to the fourth swatch.

Each swatch of hair was thereafter evaluated by a panel of 5 persons for wet and dry combing performance. In the wet combing test, a panelist combed through each swatch with the fine teeth of a comb while the hair was wet. A performance ranking of + (indicating heavy drag / difficult to comb) to ++++ (indicating low drag / easy to comb) was assigned by each panelist. The hair was then allowed to dry and a dry combing test was performed using the same rating system. The average ranking assigned by the panelists for each test is shown in Table 4.

**Table 4. Hair Manageability Panel Study - Wet/Dry Combing**

| **Conditioning Composition** | **Wet combing** | **Dry combing** |
|---|---|---|
| Inventive Composition (Example 3) | +++ | +++ |
| Control Composition (Example 4) | +++ | +++ |
| Hair Swatch without any conditioner | + | + |

| | | |
|---|---|---|
| ++++ = Best Performance + = Worst Performance | | |

The above results demonstrate that the inventive leave-on hair conditioner performed dramatically better than in the absence of a conditioner.

### Example 6

### Anti-Frizz / Humidity Study

After conducting the dry/wet combing tests described in Example 5 above, the hair swatches were placed into an environmental chamber with 85% relative humidity at 25°C for 4 hours. Each swatch of hair was thereafter evaluated by a panel of 5 persons for anti-frizz control and manageability. A performance ranking of + (indicating high frizz/difficult manageability) to ++++ (indicating smoothness/good manageability) was assigned by each panelist. The average ranking assigned by the panelists is shown in Table 5.

**Table 5. Hair Manageability Panel Study - Wet/Dry Combing**

| **Conditioning Composition** | **Friz Control** | **Manageability** |
|---|---|---|
| Inventive Composition (Example 3) | +++ | +++ |
| Control Composition (Example 4) | + | + |
| Hair Swatch without any conditioner | + | + |

| | | |
|---|---|---|
| ++++ = Best Performance + = Worst Performance | | |

The above results demonstrate that the inventive leave-on hair conditioner imparted far better friz control and manageability to the hair swatches than the control composition after exposure to relatively harsh humidity conditions.

### Example 7

### Shine Profile

The shine profiles of the first three swatches were evaluated using the SAMBA Hair system available from Bossa Nova Technologies. The swatches being one inch wide, hundreds of hair fibers are analyzed at the same time, allowing statistical and spectral signal averaging. A polarization color camera detects the scattered light reflecting from the swatch as specular or diffused light for each pixel of the image. The results of the shine profiles are shown in FIG. 1, which demonstrates that the inventive composition as prepared in Example 3 above performed better (i.e., had lower normalized units) than the control composition as prepared in Example 4 above, even at very low concentrations of the polyolefin oil blend.

## Claims

1. A hair care composition comprising:
(a) from a polyolefin oil blend comprising (i) at least one high density metallocene catalyzed polyolefin with a density above 0.90 g/cm³, (ii) at least one low density metallocene catalyzed polyolefin with a density equal to or below 0.90 g/cm³, and (iii) a cosmetically acceptable hydrocarbon oil;
(b) a thickener; and
(c) a humectant;
wherein the high and low density polyolefins have an average melt index greater than 7, wherein the polyolefin blend is present in a range of from 0.1 to 1.4 weight % by weight of the composition, and wherein the composition is substantially free of ethylene-acrylic acid copolymer.

2. The hair care composition of claim 1, wherein the pH of the composition is between 5 and 7.

3. The hair care composition of claim 1 further comprising an emollient.

4. The hair care composition of claim 1, wherein the ratio of the high density metallocene catalyzed polyolefin to the low density metallocene catalyzed polyolefin is 1:1.

5. The hair care composition of claim 1, wherein the ratio of the high density metallocene catalyzed polyolefin to the low density metallocene catalyzed polyolefin is 2:1.

6. The hair care composition of claim 1, wherein the cosmetically acceptable hydrocarbon oil is a C₁₄-C₂₂ hydrocarbon oil.

7. The hair care composition of claim 1, wherein the polyolefin oil blend has an average melt index greater than 8.0.

8. The hair care composition of claim 1, wherein the high and low density polyolefins have an average melt index greater than 8.5.

## Patentansprüche

1. Eine Haarpflegezusammensetzung, die Folgendes beinhaltet:
(a) von einer Polyolefinölmischung, beinhaltend (i) mindestens ein katalysiertes Metallocenpolyolefin hoher Dichte mit einer Dichte über 0,90 g/cm³, (ii) mindestens ein katalysiertes Metallocenpolyolefin niedriger Dichte mit einer Dichte von gleich oder unter 0,90 g/cm³, und (iii) ein kosmetisch akzeptables Kohlenwasserstofföl;
(b) ein Verdickungsmittel; und
(c) ein Befeuchtungsmittel;
wobei die Polyolefine hoher und niedriger Dichte einen durchschnittlichen Schmelzindex von größer als 7 aufweisen, wobei die Polyolefinmischung in einem Bereich von 0,1 bis 1,4 Gew.-% nach Gewicht der Zusammensetzung vorhanden ist, und wobei die Zusammensetzung im Wesentlichen frei von Ethylen-AcrylsäureCopolymer ist.

2. Haarpflegezusammensetzung gemäß Anspruch 1, wobei der pH-Wert der Zusammensetzung zwischen 5 und 7 liegt.

3. Haarpflegezusammensetzung gemäß Anspruch 1, die ferner einen Weichmacher beinhaltet.

4. Haarpflegezusammensetzung gemäß Anspruch 1, wobei das Verhältnis des katalysierten Metallocenpolyolefins hoher Dichte zu dem katalysierten Metallocenpolyolefin niedriger Dichte 1 : 1 beträgt.

5. Haarpflegezusammensetzung gemäß Anspruch 1, wobei das Verhältnis des katalysierten Metallocenpolyolefins hoher Dichte zu dem katalysierten Metallocenpolyolefin niedriger Dichte 2 : 1 beträgt.

6. Haarpflegezusammensetzung gemäß Anspruch 1, wobei das kosmetisch akzeptable Kohlenwasserstofföl ein C₁₄-C₂₂-Kohlenwasserstofföl ist.

7. Haarpflegezusammensetzung gemäß Anspruch 1, wobei die Polyolefinölmischung einen durchschnittlichen Schmelzindex von größer als 8,0 aufweist.

8. Haarpflegezusammensetzung gemäß Anspruch 1, wobei die Polyolefine hoher und niedriger Dichte einen durchschnittlichen Schmelzindex von größer als 8,5 aufweisen.

## Revendications

1. Une composition pour le soin des cheveux comprenant :
(a) à partir d'un mélange homogène de polyoléfines et d'huile comprenant (i) au moins une polyoléfine haute densité catalysée par un métallocène avec une masse volumique de plus de 0,90 g/cm³, (ii) au moins une polyoléfine basse densité catalysée par un métallocène avec une masse volumique égale ou inférieure à 0,90 g/cm³, et (iii) une huile hydrocarbonée acceptable d'un point de vue cosmétique ;
(b) un épaississant ; et
(c) un humectant ;
dans laquelle les polyoléfines haute et basse densité ont un indice de fluidité à chaud moyen supérieur à 7, dans laquelle le mélange homogène de polyoléfines est présent dans une gamme allant de 0,1 à 1,4 % en poids par poids de la composition, et la composition étant substantiellement dépourvue de copolymère d'éthylène-acide acrylique.

2. La composition pour le soin des cheveux de la revendication 1, dans laquelle le pH de la composition est entre 5 et 7.

3. La composition pour le soin des cheveux de la revendication 1 comprenant en sus un émollient.

4. La composition pour le soin des cheveux de la revendication 1, dans laquelle le rapport de la polyoléfine haute densité catalysée par un métallocène à la polyoléfine basse densité catalysée par un métallocène est de 1/1.

5. La composition pour le soin des cheveux de la revendication 1, dans laquelle le rapport de la polyoléfine haute densité catalysée par un métallocène à la polyoléfine basse densité catalysée par un métallocène est de 2/1.

6. La composition pour le soin des cheveux de la revendication 1, dans laquelle l'huile hydrocarbonée acceptable d'un point de vue cosmétique est une huile hydrocarbonée en C₁₄ à C₂₂.

7. La composition pour le soin des cheveux de la revendication 1, dans laquelle le mélange homogène de polyoléfines et d'huile a un indice de fluidité à chaud moyen supérieur à 8,0.

8. La composition pour le soin des cheveux de la revendication 1, dans laquelle les polyoléfines haute et basse densité ont un indice de fluidité à chaud moyen supérieur à 8,5.
